# EUROPEAN PATENT APPLICATION

(11) **EP 1 038 862 A2**
(43) Date of publication of application: **27.09.2000**
(21) Application number: 00105136.6
(22) Date of filing: 10.03.2000
(51) Int. Cl.: C07C 201/02

(54) **Process for the preparation of nitric monoesters of dihydroxyalkyl, dihydroxycycloalkyl and dihydroxy(poly)cycloalkyl compounds**

(30) Priority: 26.03.1999 IT MI990627
(71) Applicant: Dinamite Dipharma S.p.A., 33031 Basiliano (UD) (IT)
(72) Inventor: Beltrame, Andrea, 20021 Baranzate di Bollate (Milano) (IT); Scubla, Tiziano, 20021 Baranzate di Bollate (Milano) (IT)
(74) Representative: Minoja, Fabrizio, Dr.

(57) **Abstract**

A process for the preparation of nitric monoesters of dihydroxyalkyl, dihydroxycycloalkyl or dihydroxy(poly)cycloalkyl compounds starting from the respective nitric diesters of dihydroxyalkyl, dihydroxycycloalkyl or dihydroxy(poly)cycloalkyl compounds, which comprises the hydrogenation of the nitric diesters with a platinum (0) catalyst.

## Description

The present invention generally relates to the organic chemistry field.

More particularly, the invention relates to a process for the preparation of nitric monoesters of dihydroxyalkyl, dihydroxycycloalkyl and dihydroxy(poly)cycloalkyl compounds by transformation of the nitric diesters of said compounds into the corresponding nitric monoesters.

Nitric monoesters of dihydroxyalkyl, dihydroxycycloalkyl and dihydroxypolycycloalkyl compounds are a class of compounds including products such as isosorbide mononitrate, which are used in the pharmaceutical field for the treatment of cardiovascular diseases, in particular coronary diseases and angina pectoris (Biopharm. And Drug Disp. 1981, 2, 255-263).

The preparation of said nitric monoesters cannot be carried out by direct nitration (Chemistry and Technology of Explosives 1965, vol. 2, PWN Warzawa), but it is effected by reaction of the corresponding monohaloderivatives with silver nitrate (Synth. Comm. 1992, 22, 677) or, alternatively, by bis-acylation followed by monohydrolysis, nitration and final cleavage of the second acyl group (US 4 065 488; EP-A-0 045 076).

A more interesting approach from the industrial point of view is that comprising the dinitration of the dihydroxylic compound and the subsequent reductive transformation of the resulting dinitroester into monoester.

Hydrazine hydrate has been suggested as the reducing agent (FR-A-8103906) but said reactive is problematic for the industrial use due to its ascertained cancerogenicity.

Alternatively to hydrazine hydrate, the use of a reducing consisting system of metals and weak acids has been proposed, in particular zinc and acetic acid or a system consisting of hydrogen and a palladium on charcoal catalyst (EP-A-0 201 067, Gazz. Chim. It. 1987, 117, 173).

The above cited reducing systems have, however, the drawback that not only the nitric monoester is formed, but also a significant amount of the dihydroxylic compound, which makes the purification of the desired nitric monoester difficult.

Furthermore, a drawback of the process using zinc and acetic acid is that zinc is used in amounts equivalent to or higher than the stoichiometric, which involves the production of wastes with high amounts of zinc salts to be disposed of.

The present invention aims at providing a process for the selective reduction of nitric diesters of dihydroxyalkyl, dihydroxycycloalkyl and dihydroxy(poly)cycloalkyl compounds to the respective nitric monoesters.

This problem has been solved, according to the invention, by using a reducing system based on hydrogen and a platinum (0) catalyst.

Dihydroxyalkyl compounds nitric diesters for use in the process of the invention are those generally containing a straight or branched C₃-₂₀alkyl residue, optionally containing heteroatoms and/or having one or more substituents selected from the group consisting of C₁₋₈alkoxy, C₁₋₈alkylcarboxy, carboxamido, nitro, cyano, halogen.

Dihydroxycycloalkyl compounds nitric diesters for use in the process of the invention are generally those containing a C₅₋₇cycloalkyl residue, optionally containing heteroatoms and/or having one or more substituents selected from the group consisting of C₁₋₅alkyl, C₅₋₇cycloalkyl, C₁₋₈alkoxy, C₁₋₈alkylcarboxy, carboxamido, nitro, cyano, halogen.

Dihydroxy(poly)cycloalkyl compounds nitric diesters for use in process of the invention are generally those containing a polycyloalkyl residue deriving from the fusion of 5-7 membered cycles, optionally containing heteroatoms and/or having one or more substituents selected from the group consisting of C₁₋₅alkyl, C₅₋₇cycloalkyl, C₁₋₈alkoxy, C₁₋₈alkylcarboxy, carboxamido, nitro, cyano, halogen.

The platinum (0) catalyst used in the process of the invention is generally selected from the group consisting of elemental platinum, supported platinum, platinum complexes with ligands such as acetylacetonate, benzonitrile and triphenylphosphine, platinum salts (II) which can be reduced to platinum (0).

Said platinum (0) catalyst is preferably platinum on charcoal.

The platinum (0) catalyst is preferably used in amounts equivalent to 0.001 - 0.01 mols of platinum per mol of nitric diester, conveniently in amounts equivalent to 0.003 - 0.007, preferably 0.005, mols of platinum per mol of nitric diester.

The catalytic hydrogenation of the process of the invention is carried out preferably in a polar solvent selected from the group consisting of optionally alkoxysubstituted C₁₋₃alcohols, C₃₋₅ketones and mixtures of said alcohols and ketones with water.

Said polar solvent is advantageously selected from methanol, ethanol, isopropanol, 2-methoxy-1-propanol, acetone, methyl ethyl ketone and mixtures thereof with water.

The catalytic hydrogenation is generally carried out at temperatures ranging from 0° to 50°C, preferably 20-30°C, and under a hydrogen pressure ranging from 0.1 to 50 bars, preferably 20-35 bars.

The nitric monoester is generally obtained by filtration of the catalyst from the reaction mixture, evaporation of the solvent under vacuum, dilution of the residue with water, separation of unreacted nitric diester, extraction of the nitric monoester with solvent and final purification (for example by crystallization from a suitable solvent, distillation, column chromatography etc.).

The process according to the present invention will be further described with reference to the following examples.

### EXAMPLE 1

104 g of 1,5-pentanediol (1 mol) were added in portions, in 15 minutes and at 10-15°C, to a mixture of sulfuric acid (300 g) and fuming nitric acid (300 g). The mixture was kept under stirring to complete the reaction, then stirring was interrupted. The product was separated from the acidic phase, washed with water and then with a 5% bicarbonate aqueous solution, to obtain 1,5-pentanediol nitric diester, which was used directly in the subsequent reaction (illustrated in example 2).
¹H NMR (200 MHz, CDCl3) δ in ppm: 1.38 (3H, J = 6.2 Hz); 1.40-1.65 (m, 2H); 1.55-1.80 (m, 4H); 4.48 (t, 2H, J = 6.3 Hz); 5.08 (m, 1H, J = 6.2 Hz).

### EXAMPLE 2

A 1 l hydrogenation reactor was loaded with 100 g of 1,5-pentanediol nitric diester (0.67 mols), 600 g of acetone and 9 g (2.3 mmoles) of 5% platinum on charcoal. The reactor was kept under hydrogen atmosphere under a pressure of 30-35 bars, with stirring and at a temperature of 20-25°C for 150 minutes. The mixture was then filtered and the solvent was evaporated off under vacuum and replaced with water. After separation of the aqueous phase from the organic one (which contained unreacted nitric diester) the aqueous phase was extracted with ethyl ether. The ether phase was dried over sodium sulfate and the solvent was evaporated off under vacuum to obtain 67 g of 1,5-pentanediol nitric monoester.
¹H NMR (200 MHz, CDCl3) δ in ppm: 1.48-1.80 (m, 6H); 2.60 (s, 1H); 3.60 (t, 2H); 4.50 (t, 1H).

### EXAMPLE 3

A 1 l hydrogenation reactor was loaded with isosorbide dinitrate (177.5 g, 758.5 mmoles) (compound of formula (I)), 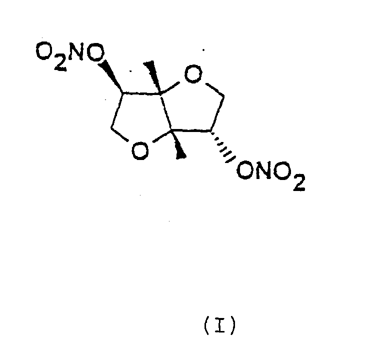 butyl acetate (533 g) and 5% platinum on charcoal (9 g, 2.3 mmoles). The reactor was kept under hydrogen atmosphere under a pressure of 4 bar, with stirring and at a temperature of 20°C for 150 minutes. The mixture was filtered, then added with sulfuric acid (31.6 g) and kept under vacuum for 120 minutes. The mixture was then added with 30% sodium hydroxide to reach pH = 5. The solvent was evaporated off under vacuum and replaced with water. The precipitated isosorbide dinitrate was filtered and that remaining in solution was extracted with toluene. The aqueous solution was extracted with butyl acetate and the organic phase was concentrated under vacuum to give a 20-25% isosorbide-5-mononitrate solution. The solution was cooled to 5-10°C and the crystallized product was filtered and dried under vacuum at 50°C, thereby obtaining 38 g of isosorbide-5-mononitrate, (compound of formula (II)) m.p. = 87-9°C.

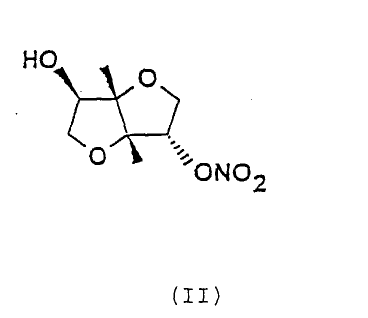

It is clear from the example that, when the starting nitric diester is an asymmetric compound, the process of the present invention selectively yields only one isomer.

## Claims

1. A process for the preparation of nitric monoesters of dihydroxyalkyl, dihydroxycycloalkyl or dihydroxy(poly)cycloalkyl compounds starting from the respective nitric diesters of dihydroxyalkyl, dihydroxycycloalkyl or dihydroxy(poly)cycloalkyl compounds, which comprises the hydrogenation of the nitric diesters with a platinum (0) catalyst.

2. A process as claimed in claim 1, wherein said dihydroxyalkyl compounds nitric diesters contain a straight or branched C₃₋₂₀alkyl residue, optionally containing heteroatoms and/or having one or more substituents selected from the group consisting of C₁₋₈alkoxy, C₁₋₈alkylcarboxy, carboxamido, nitro, cyano, halogen; said dihydroxycycloalkyl compounds nitric diesters contain a C₅₋₇cycloalkyl residue optionally containing heteroatoms and/or having one or more substituents selected from the group consisting of C₁₋₅alkyl, C₅₋₇cycloalkyl, C₁₋₈alkoxy, C₁₋₈alkylcarboxy, carboxamido, nitro, cyano, halogen; and said dihydroxy(poly)cycloalkyl compounds nitric diesters contain a polycyloalkyl residue deriving from the fusion of 5-7 membered cycles optionally containing heteroatoms and/or having one or more substituents selected from the group consisting of C₁₋₅alkyl, C₅₋₇cycloalkyl, C₁₋₈alkoxy, C₁₋₈alkylcarboxy, carboxamido, nitro, cyano, halogen.

3. A process as claimed in claim 1 or 2, wherein said platinum (0) catalyst is selected from the group consisting of elemental platinum, supported platinum, platinum complexes with ligands selected from acetylacetonate, benzonitrile and triphenylphosphine, platinum (II) salts which can be reduced in situ to platinum (0).

4. A process as claimed in claim 3, wherein said platinum catalyst is platinum on charcoal.

5. A process according to any one of the above claims, wherein said catalyst is used in amounts equivalent to 0.001 - 0.01 mols of platinum per mol of nitric diester.

6. A process as claimed in claim 5, wherein said catalyst is used in amounts equivalent to 0.003 - 0.007, preferably 0.005, mols of platinum per mol of nitric diester.

7. A process according to any one of the above claims, wherein said catalytic hydrogenation is carried out in a polar solvent selected from the group consisting of optionally alkoxy-substituted C₁₋₃alcohols, C₁₋₃ketones and mixtures of said alcohols and ketones with water.

8. A process as claimed in claim 7, wherein said polar solvent is selected from methanol, ethanol, isopropanol, 2-methoxy-1-propanol, acetone, methyl ethyl ketone and mixtures thereof with water.

9. A process according to any one of the above claims, wherein said catalytic hydrogenation is carried out at temperatures ranging from 0° to 50°C and under a hydrogen pressure ranging from 0.1 and 50 bars.

10. A process as claimed in claim 9, wherein said catalytic hydrogenation is carried out at a temperature of 20°-30°C and under a hydrogen pressure of 20 - 35 bars.
